# EUROPÄISCHE PATENTSCHRIFT

(11) **EP 4 171 409 B1**
(45) Veröffentlichungstag und Bekanntmachung des Hinweises auf die Patenterteilung: **10.09.2025**
(21) Anmeldenummer: 21739581.3
(22) Anmeldetag: 23.06.2021
(51) Int. Cl.: A61B 17/70, A61B 17/86, A61B 17/88, F16B 35/06, F16B 23/00

(54) **WERKZEUGANSATZSTELLE MIT AUSRICHTHILFE FÜR SCHRAUBENELEMENTE**
TOOL APPLICATION POINT WITH ORIENTATION AID FOR SCREW ELEMENTS
EMPLACEMENT D'INSERTION D'OUTIL ÉQUIPÉ D'UNE AIDE À L'ORIENTATION POUR ÉLÉMENTS VIS

(30) Priorität: 24.06.2020 DE 102020003774
(43) Veröffentlichungstag der Anmeldung: 03.05.2023
(73) Patentinhaber: Mimeo Medical GmbH, 70794 Filderstadt (DE)
(72) Erfinder: HEUER, Frank, 70794 Filderstadt (DE)
(74) Vertreter: Stolmár & Partner Patentanwälte PartG mbB
(86) Internationale Anmeldenummer: PCT/EP2021/067103
(87) Internationale Veröffentlichungsnummer: WO 2021/259978

(56) Entgegenhaltungen:
- EP-A1- 0 626 277
- EP-A1- 2 932 929
- DE-A1- 102013 105 744
- US-A- 5 171 117

## Beschreibung

### Stand der Technik

Zum effizienten Operieren zählt vor allem das Einsparen von Zeit, was durch unterschiedliche Vereinfachungen der Anwendung erzielt werden kann. Hierzu zählt insbesondere das Zusammenfügen einer Schraube mit einem Schraubendreherinstrument, da dies einer der häufigsten Schritte in einer OP ist. Ein wesentlicher Schritt beim Ansetzen eines Schraubendreherinstruments mit einer Schraube ist es, dass der Werkzeugantrieb des Schraubendreherinstruments genau zur rotatorischen Ausrichtung der Werkzeugansatzstelle der Schraube passen muss. Dies gelingt nicht immer auf Anhieb und ist für den Anwender nicht intuitiv. Dadurch kann dieser Schritt, auch durch die hohe Anzahl von Wiederholungen in einer OP, enorm zeitraubend sein. Sollte eine OP unter schweren Sichtbedingungen bzw. als minimalinvasiver Eingriff durchgeführt werden, kann sich das Andocken des Schraubendreherinstruments an die Schraube teilweise auch fehlerbehaftet sein.

Aus EP2932929A1 ist ein Schraubenelement bekannt, welches einen Torx^{®}-ähnlichen Vielzahnrund als Werkzeugansatzstelle zum Antreiben einer Schraube besitzt. Damit ein Schraubendreherinstrument in einer beliebigen rotatorischen Position mit dem Schraubenelement zusammengefügt werden kann, befinden sich am distalen Bereich der Werkzeugansatzstelle an jedem Zahnprofil symmetrisch angeordnete kegelartige Führungsnuten. Nachteilig ist allerdings, dass durch diese Führungsnuten die Kontaktflächen der ineinandergreifenden Zähne zwischen Schraubendreherinstrument und Schraube verkleinert sind, denn dort wo die Führungsnuten vorgesehen sind, fehlt der Kontakt zum Schraubendreher. Damit sind im Vergleich zu einer Werkzeugansatzstelle ohne Führungsnuten und gleicher Bauhöhe deutlich geringere Drehmomente übertragbar. Dies ist für orthopädische Schraubenelemente von großem Nachteil, da häufig hohe Drehmomente übertragen werden müssen.

Die US 5 171 117 A offenbart eine Befestigungsvorrichtung 10 mit einem Kopf- 16 und einem Gewindeabschnitt 18. Iin dem Kopf ist ein Sitz 20 für ein Werkzeug ausgebildet, der gekürzte Sitznasen (*"truncated socket lobes*") aufweist und auf diese Weise Führungsflächen bereitstellt. Diese dienen dazu, ein verfrühtes Eingreifen des Werkzeugs mit den Sitznasen zu vermeiden

Mit der hier dargestellten Erfindung ist es möglich genauso hohe Einschraub-Drehmomente, wie sie mit einem Vielzahnrund ohne Ausrichthilfe möglich sind, zu erzielen. Dies wird dadurch erreicht, indem die Ausrichthilfe unsymmetrisch im Zahnprofil vorgesehen ist. Aus den Grundlagen der Mechanik ist bekannt, dass das Drehmoment zum Einschrauben einer Schraube immer höher ist, als das Drehmoment zum Ausschrauben einer Schraube. Deshalb ist es notwendig, die Kontaktflächen einer Werkzeugansatzstelle zu einem Schraubendreher in Eindreh-Richtung zu maximieren, wobei gleichzeitig die gegenüberliegenden Kontaktflächen in Ausdreh-Richtung kleiner vorgesehen sein können. Die verkleinerten Kontaktflächen in Ausdreh-Richtung ermöglichen Bauraum für eine entsprechende Ausrichthilfe für das Schraubendreherinstrument.

### Darstellung der Erfindung

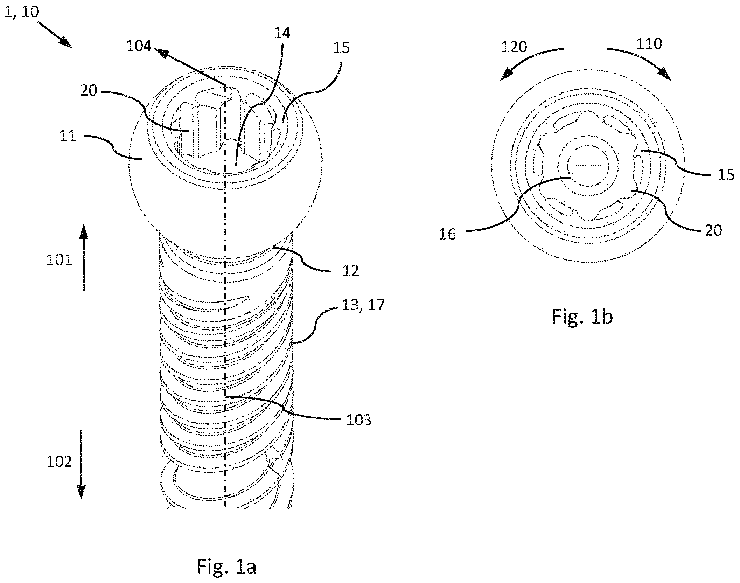

Für das erfindungsgemäße Schraubenelement (10), sind raumzuweisende Koordinatenreferenzen definiert, wie zum Beispiel die proximale Richtung (101), die distale Richtung (102), die sich entlang einer Zentralachse (103) ausdehnen. Von der Zentralachse (103) nach außen abgehend definiert sich die radiale Ausbreitung (104). Die umfängliche Ausbreitung ist durch einen konstanten Radius und entlang eines variablen Umfangswinkels definiert (Fig. 1a).

Verwendet wird das erfindungsgemäße Schraubenelement (10) für die Fixation von Knochenkomponenten und Knochenfragmenten. Es besteht dabei aus einem Schaft (13) mit einem Außengewinde (17) und eine von radial innen ausgehend Werkzeugansatzstelle (20). Das Außengewinde gibt dabei einen Drehsinn für das Einschrauben und Ausschrauben des Schraubenelements (10) vor. In Abhängigkeit dieses Drehsinns sind zwei weitere Raumrichtungen definiert; die Einschraub-Richtung (110) und die Ausschraub-Richtung (120) (Fig. 1b).

Das Schraubenelement kann eine Knochenschraube mit einem Kopf sein, der einen Antriebsabschnitt umfasst, welcher hier als Werkzeugansatzstelle (20) definiert ist. Es kann sich jedoch auch um eine Madenschraube handeln, die als Verriegelungselement im Aufnahmeteil einer polyaxialen Pedikelschraube oder in einer Knochenplatte verwendet wird. Allgemeiner kann das Schraubenelement in Fällen mit schlechter oder fehlender Sicht auf die Einsatzstelle verwendet werden, in denen die Position einer bereits platzierten Schraube angepasst werden muss. In einer bevorzugten Ausführungsform besitzt das Schraubenelement (10) einen Kopf (11), welcher sphärisch ausgeformt ist, einen Halsbereich (12) und einen Schaft-Bereich (13) mit Knochengewinde (18) und die Werkzeugansatzstelle (20) wird im Kopf (11) bereitgestellt (Fig. 1a). Die Werkzeugansatzstelle (20) ist in proximaler Richtung (101) offen und mündet in einer konzentrischen kegelartigen Ausnehmung (15). Der Kopf (11) ist vorzugsweise als Linse, Schrägkopf oder als Kugelkopf ausgebildet. Es sind aber auch eine Zusammensetzung aus unterschiedlichen Rundungen und Flächen denkbar. Hauptmerkmal des Kopfes ist es, dass der Kopf (11) einen größeren Außendurchmesser als der Halsbereich (12) aufweist. Zur minimal-invasiven Versorgung ist es vorteilhaft, wenn der Knochenanker eine vollständig durchquerende Kanülierungsöffnung (16) besitzt, durch die ein chirurgischer Führungsdraht geführt werden kann. Vorzugsweise ist die Werkzeugansatzstelle (20) als Sacklock ausgebildet und nach distaler Richtung (102) von einer Wandung (14) begrenzt ist. Optional kann diese Wandung (14) als Schräge ausgebildet sein, die kegelartig in zunehmender distaler Richtung (102) nach radial innen verläuft.

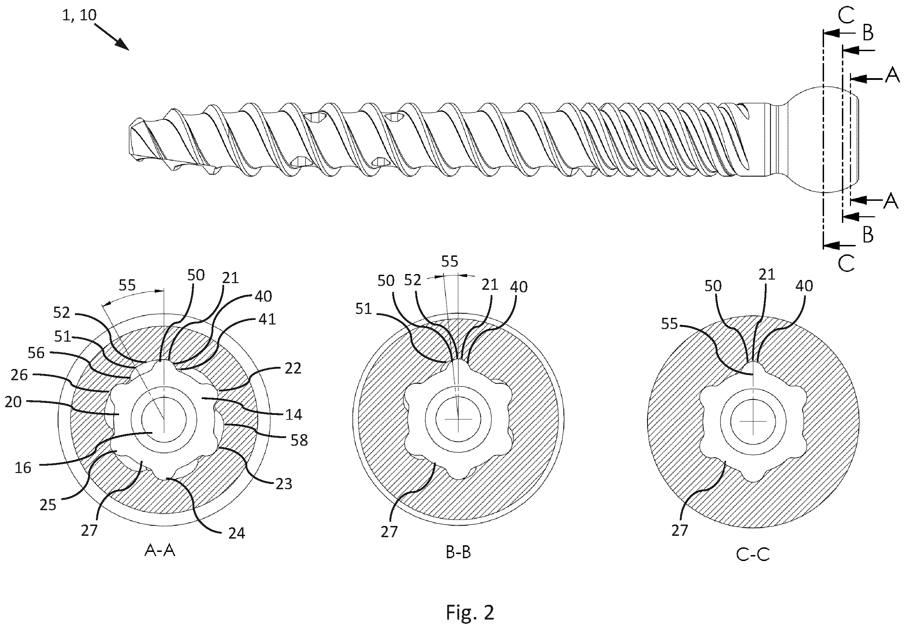

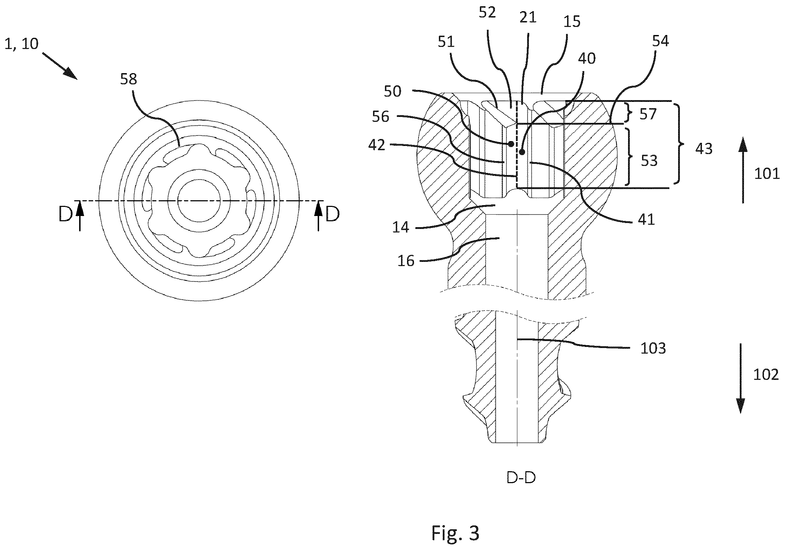

Ergänzend ist zu erwähnen, dass das Schraubenelement (10) eine von radial innen ausgehende Werkzeugansatzstelle (20) besitzt und darin eine zentrale Öffnung (27) vorgesehen ist. In der Wand dieser Öffnung (27) sind mindestens fünf nach radial außen (104) und hauptsächlich parallel zur Schraubenachse (103) gerichtete Zahnprofile (z.B. 21, 22, 23, 24, 25, 26) ausgebildet (Fig. 2). Vorzugsweise sind die Zahnprofile als konkave Wandungen gestaltet, so dass sich ein Torx^{®}-ähnliches oder genormtes Torx^{®}-Profil ergibt. Optimalerweise befinden sich Übergangsradien (56, 41) zwischen den Zahnprofilen (z.B. 21) und der zentralen Öffnung (27). Für jedes Zahnprofil kann die Zahnprofilfläche (z.B. 21) eine Eindreh-Wandung (40) in Eindreh-Richtung (110) und eine Ausdreh-Wandung (50) in Ausdreh-Richtung (120) unterteilt werden. Zur Unterteilung dieser Wandungen (40 und 50) dient eine gedachte Trennlinie (42) in Fig. 3. Ein wesentliches Merkmal der Erfindung ist, dass die Fläche der Eindreh-Wandung (40) größer ist als die Fläche der Ausdreh-Wandung (50). Somit hat ein Schraubendreher (60) mit einem komplementären Zahnprofil die volle Eingriff-Höhe in Eindreh-Richtung. In die weniger belastete Ausdreh-Richtung ist die Kontaktfläche zwischen Schraubendreherinstrument und Schraubenelement kleiner.

In Fig. 3 sind die erfindungsgemäßen Merkmale dargestellt. Zu erkennen ist, dass die Werkzeugansatzstelle (20) entlang der longitudinalen Achse (103) in mindestens zwei Abschnitte (53, 57) einteilbar ist, wobei von distaler Richtung kommend ein erster Abschnitt (53) ausgebildet ist und bis zur Höhe einer Abschnittstrennebene (54) die Flächen der Eindreh- und Ausdreh-Wandungen (40, 50) von jedem Zahnprofil (z.B. 21...26) in etwa gleich groß sind. Des Weiteren verläuft die Eindreh-Wandung (40) auf gesamter Höhe beider Abschnitte (43, 53 und 57) hauptsächlich parallel zur Schraubenachse (103). Im zweiten Abschnitt (57) ist eine Führungs-Wandung (51) ausgehend von der Abschnittstrennebene (54) ausgebildet, wobei die Führungs-Wandung (51) an die Ausdreh-Wandung (50) des ersten Abschnitts (53) ansetzt und diese Führungs-Wandung (51) in proximaler Richtung (101) von der Eindreh-Wandung (40) zunehmend beabstandet ist. Die zunehmende Beabstandung zwischen der Führungs-Wandung (51) und Eindreh-Wandung (40) verläuft hauptsächlich entlang des Umfangs in Ausdreh-Richtung (120) und dadurch ergibt sich eine in Umfangsrichtung erstreckende Wandung (52).

Zusammengefasst heißt dies, dass im ersten Abschnitt (53) eine herkömmliche Werkzeugansatzstelle vorgesehen ist, die symmetrische und hauptsächlich parallel zur Schraubenachse (103) ausgerichtete Zahnprofile besitzt (z.B. 21...26). Im zweiten Abschnitt (57) sind die Wandungen so angeordnet, dass sie die Ausrichthilfe für das Schraubendreherinstrument (60) bereitstellen. Die Ausrichtunghilfe ist in Fig. 2 im Schnitt quer zur Schraubachse (103) auf Höhe des zweiten Abschnitts (57) dargestellt. Dabei ist zu erkennen, dass die Wandungen (51, 52 und 40) eine Schnitt-Kontur erzeugen, die näherungsweise einem rotatorischen Langloch (58) entspricht und durch einen Öffnungswinkel (55) definierbar ist. Der Öffnungswinkel (55) besitzt einen maximalen Winkel zwischen 10° bis 60°, vorzugsweise jedoch 20° bis 50°. Dieser Winkel (55) verkleinert sich von proximaler Richtung (101) nach distaler Richtung (102), wobei dieser Winkel (55) im ersten Abschnitt (53) konstant bleibt. Diese Merkmale sind anhand der unterschiedlichen Schnittebenen in Fig. 2 illustriert.

Vorzugsweise besitzen die Abschnitte (53 und 57) eine unterschiedliche Höhe. Denkbar sind beispielsweise; dass die Höhe des ersten Abschnitts (53) größer ist als die Höhe für den zweiten Abschnitt (57), oder die Höhe des zweiten Abschnitts (57) größer ist als die Höhe für den ersten Abschnitt (53), oder auch dass die Höhen der beiden Abschnitte (53) und (57) in etwa gleich sind.

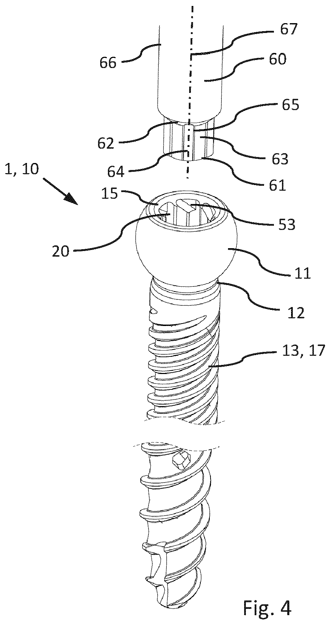

In Fig. 4 dargestellt ist ein typisches Schraubendreherinstrument (60) mit einem Schaft (66), welcher eine longitudinale Schraubendreherachse (67) definiert. Am distalen Ende (61) befindet sich eine Antriebseinheit (65) mit Zähnen (64) und einem Kern (63). Die Antriebseinheit (65) mündet in proximaler Richtung in einem kegelartigen Auslauf (62). Die bereits erwähnten Abschnitte (53, 57) der Werkzeugansatzstelle (20) des Schraubenelements (10) münden in eine kegelartige konzentrische Ausnehmung (15) in proximaler Richtung (101) (Fig. 4). Die kegelartige Ausnehmung (15) hat zwei Funktionen. Einerseits werden bei Erstkontakt des Schraubendreherinstruments (60) mit dem Schraubenelement (10) die Schraubenachse (103) mit der Achse (67) des Schraubendreherinstruments (60) orthograd zueinander ausgerichtet indem das distale Ende (61) entlang des Konus (15) zur Zentralachse (103) des Schraubenelements (10) geführt wird. Anderseits wird bei voll eingeführtem Schraubendreherinstrument (60) ein flächiger Kontakt zwischen der kegelartigen Ausnehmung (15) und dem kegelartigen Auslauf (62) hergestellt, der für eine belastbare orthograde Ausrichtung des Schraubenelements (10) im Bezug zum Schraubendreherinstrument (60) sorgt.

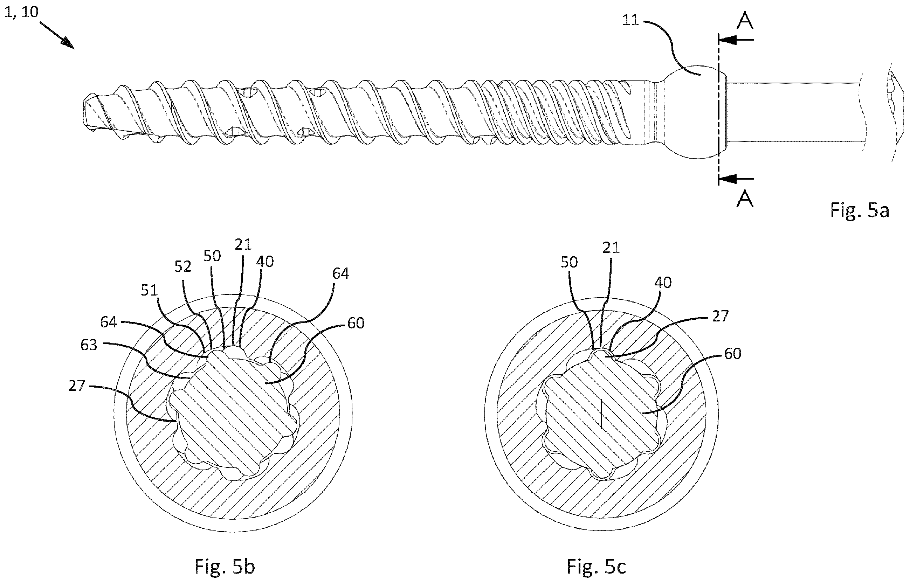

Ein weiteres Merkmal ist, dass das Schraubendreherinstrument (60) eine dem ersten Abschnitt (53) komplementär ausgebildete Antriebseinheit (65) mit Zähnen (64) besitzt (Fig. 5c), welche sich hauptsächlich parallel der Schraubendreher-Zentralachse (67) erstreckt und das Schraubenelement (10) im Bereich des zweiten Abschnitts (57) Führungswandungen (51) besitzt, welche die Zähne (64) des Schraubendreherinstruments (60) rotatorisch um die Schraubendreher-Zentralachse (67) ausrichten (Fig. 5b), so dass für die Zähne (64) des Schraubendreherinstruments (60) eine größere Kontaktfläche in Eindreh-Richtung (110) als in Ausdreh-Richtung (120) bereitgestellt wird.

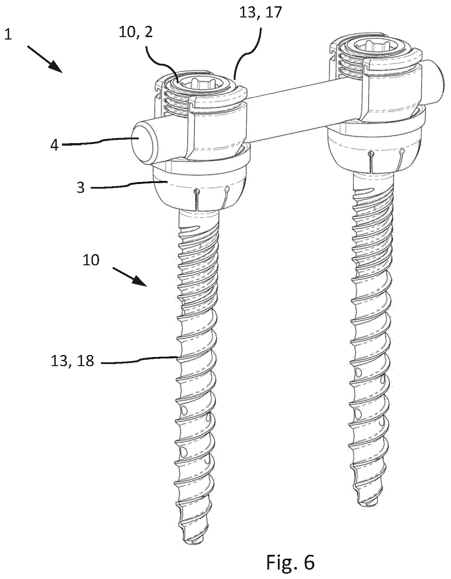

Fig. 6 zeigt ein System einer Osteosynthesevorrichtung (1) für die Behandlung von Wirbelsäulendeformitäten und -defekten, welches aus mindestens zwei Schraubenelementen (10) besteht, wobei ein Schraubenelement (10) als Knochenanker mit einem Knochengewinde (18) vorgesehen ist, welcher polyaxial in einem u-förmigen Gabelkopf gelagert ist (3) und ein zweites Schraubenelement (2) als Verschlusselement zur Fixierung eines Verbindungsstab (4) geeignet ist.

### Kurze Beschreibung der Zeichnungen zeigen

Fig. 1a eine Schrägansicht des erfindungsgemäßen Schraubenelements.
Fig. 1b die Draufsicht auf die Werkzeugansatzstelle.
Fig. 2 zeigt eine Seitenansicht des erfindungsgemäßen Schraubenelements und drei an unterschiedlicher Position vorgesehene Schnitte durch die Werkzeugansatzstelle.
Fig. 3 illustriert einen Schnitt in der Seitenansicht der Werkzeugansatzstelle.
Fig. 4 stellt das Zusammenspiel aus Schraubenelement und Schraubendreherinstrument dar.
Fig. 5a zeigt eine Seitenansicht gemäß Fig. 4.
Fig. 5b zeigt im Schnitt, wie das Schraubendreherinstrument beim Einsetzen in die Werkzeugansatzstelle in einer beliebigen rotatorischen Position geführt und ausgerichtet wird, und
Fig. 5c zeigt den Zustand beim Einsetzen des Schraubendreherinstruments, sobald die Antriebseinheit des Schraubendreherinstruments den Abschnitt zur Ausrichtung passiert hat und im Eingriff mit dem Vielzahnrund ist.
Fig. 6 illustriert unterschiedliche Schraubenelemente einer Osteosynthesevorrichtung.

## Patentansprüche

1. Schraubenelement (10) für die Fixation von Knochenkomponenten und Knochenfragmenten bestehend aus einem Schaft (13) mit einem Außengewinde (17) und eine sich am Schaft (13) entlang erstreckende longitudinalen Achse (103) und dadurch eine distale (102) und eine proximale (101) Richtung definiert, sowie sich eine Eindreh-Richtung (110) und eine davon entgegengesetzte Ausdreh-Richtung (120) ergibt, und von radial innen ausgehend eine Werkzeugansatzstelle (20) besitzt und die Werkzeugansatzstelle (20) eine zentrale Öffnung (27) besitzt und in der Wand dieser Öffnung (27) mindestens fünf nach radial außen (104) und hauptsächlich parallel zur Schraubenachse (103) gerichtete Zahnprofile (21, 22, 23, 24, 25, 26) ausgebildet sind, und sich für jedes Zahnprofil eine Eindreh-Wandung (40) in Eindreh-Richtung (110) und eine Ausdreh-Wandung (50) in Ausdreh-Richtung (120) unterteilen lässt, wobei die Werkzeugansatzstelle (20) entlang der longitudinalen Achse (103) in mindestens zwei Abschnitte einteilbar ist, wobei von distaler Richtung kommend ein erster Abschnitt (53) ausgebildet ist und bis zur Höhe einer Abschnittstrennebene (54) die Flächen der Eindreh- und Ausdreh-Wandungen (40, 50) von jedem Zahnprofil (21, 22, 23, 24, 25, 26 ) in etwa gleich groß sind und die Fläche der Eindreh-Wandung (40) größer als die Fläche der Ausdreh-Wandung (50) ist
**dadurch gekennzeichnet, dass**
die Werkzeugansatzstelle (20) in proximaler Richtung (101) offen ist und in einer konzentrischen kegelartigen Ausnehmung (15) mündet und die Werkzeugansatzstelle (20) entlang longitudinaler Richtung (103) in mindestens zwei Abschnitte einteilbar (53, 57) ist und die Eindreh-Wandung (40) auf gesamter Höhe beider Abschnitte (43, 53, 57) hauptsächlich parallel zur Schraubenachse (103) verläuft.

2. Schraubenelement (10) nach Anspruch 1, **dadurch gekennzeichnet, dass** im zweiten Abschnitt (57) eine Führungs-Wandung (51) ausgehend von der Abschnittstrennebene (54) ausgebildet ist, wobei die Führungs-Wandung (51) an die Ausdreh-Wandung (50) des ersten Abschnitts (53) ansetzt und diese Führungs-Wandung (51) in proximaler Richtung (101) von der Eindreh-Wandung (40) zunehmend beabstandet ist.

3. Schraubenelement (10) nach Anspruch 2, **dadurch gekennzeichnet, dass** die zunehmende Beabstandung zwischen der Führungs-Wandung (51) und Eindreh-Wandung (40) hauptsächlich entlang des Umfangs in Ausdreh-Richtung (120) verläuft und sich dadurch eine in Umfangsrichtung erstreckende Wandung (52) ergibt.

4. Schraubenelement (10) nach einem der vorhergehenden Ansprüche, **dadurch gekennzeichnet, dass** im Schnitt quer zur Schraubachse (103) auf Höhe des zweiten Abschnitts (57) die Wandungen (51, 52, 40) eine Schnitt-Kontur besitzen, die näherungsweise einem rotatorischen Langloch (58) entspricht und durch einen Öffnungswinkel (55) definierbar ist.

5. Schraubenelement (10) nach einem der vorhergehenden Ansprüche, **dadurch gekennzeichnet, dass** der Öffnungswinkel (55) einen maximalen Winkel zwischen 10° bis 60°, vorzugsweise jedoch 20° bis 50° besitzt, und sich dieser Winkel (55) nach distaler Richtung (102) verkleinert.

6. Schraubenelement (10) nach einem der vorhergehenden Ansprüche, **dadurch gekennzeichnet, dass** die Höhe des ersten Abschnitts (53) größer ist als die Höhe für den zweiten Abschnitt (57), dass die Höhe des zweiten Abschnitts (57) größer ist als die Höhe für den ersten Abschnitt (53) oder die Höhen der beiden Abschnitte (53) und (57) in etwa gleich sind.

7. Schraubenelement (10) nach einem der vorhergehenden Ansprüche, **dadurch gekennzeichnet, dass** die Werkzeugansatzstelle (20) nach distaler Richtung (102) von einer Wandung (14) begrenzt ist.

8. Schraubenelement (10) nach Anspruch 7, **dadurch gekennzeichnet, dass** die Wandung (14) als Schräge in zunehmender distaler Richtung (102) nach radial innen verläuft.

9. Schraubenelement (10) nach einem der vorhergehenden Ansprüche, **dadurch gekennzeichnet, dass** die zentrale Öffnung (27) einen konzentrisch zylindrischen Verlauf besitzt.

10. Schraubenelement (10) nach einem der vorhergehenden Ansprüche, **dadurch gekennzeichnet, dass** das Schraubenelement (10) zusätzlich einen Kopf (11), einen Halsbereich (12) und einen Schaft-Bereich (13) mit Knochengewinde (18) aufweist und die Werkzeugansatzstelle (20) im Kopf (11) bereitgestellt wird.

11. Schraubenelement (10) nach einem der vorhergehenden Ansprüche, **dadurch gekennzeichnet, dass** das Schraubenelement (10) eine durchgängige Kanülierung (16) besitzt.

12. System einer Osteosynthesevorrichtung (1), **dadurch gekennzeichnet, dass** die Osteosynthesevorrichtung (1) aus mindestens zwei Schraubenelementen (10) nach einem der vorhergehenden Ansprüche besteht, wobei ein Schraubenelement (10) als Knochenanker mit einem Knochengewinde (18) vorgesehen ist und ein zweites Schraubenelement (2) als Verschlusselement zur Fixierung eines Verbindungsstabs (4) geeignet ist.

13. System bestehend aus einem Schraubendreherinstrument (60) und mindestens einem Schraubenelement (10), nach einem der vorhergehenden Ansprüche, **dadurch gekennzeichnet, dass** das Schraubendreherinstrument (60) eine dem ersten Abschnitt (53) komplementär ausgebildete Antriebseinheit (65) mit Zähnen (64) besitzt, welche sich hauptsächlich parallel der Schraubendreher-Zentralachse (67) erstreckt und das Schraubenelement (10) im Bereich des zweiten Abschnitts (57) Führungswandungen (51) besitzt, welche die Zähne (64) des Schraubendreherinstruments (60) rotatorisch um die Schraubendreher-Zentralachse (67) ausrichten, so dass für die Zähne (64) des Schraubendreherinstruments (60) eine größere Kontaktfläche in Eindreh-Richtung (110) als in Ausdreh-Richtung (120) bereitgestellt wird.

## Claims

1. Screw element (10) for the fixation of bone components and bone fragments comprising a shaft (13) with an outside screw thread (17) and a longitudinal axis (103) extending along the shaft (13) and thereby defining a distal direction (102) and a proximal direction (101), and it results in an insertion direction (110) and an opposite removal direction (120), and has a tool attachment point (20) starting radially inwards and the tool attachment point (20) has a central opening (27) and in the wall of this opening (27) at least five tooth profiles (21, 22, 23, 23) directed radially outwards (104) and mainly parallel to the screw axis (103) are formed in the wall of this opening (27), and for each tooth profile an insertion wall (40) in insertion direction (110) and a removal wall (50) in removal direction (120) can be classified, wherein the tool attachment point 20) can be divided into at least two sections along the longitudinal axis (103), wherein, coming from the distal direction, a first section (53) is formed and, up to the level of a section separating plane (54), the surfaces of the insertion and removal walls (40, 50) of each tooth profile (21, 22, 23, 23, 24, 25, 26) are approximately equal and the surface of the insertion wall (40) is greater than the surface of the removal wall (50) **characterized in that**
the tool attachment point (20) is open in the proximal direction (101) and ends into a concentric conical recess (15) and the tool attachment point (20) can be divided into at least two sections (53, 57) along the longitudinal direction (103) and the insertion wall (40) runs mainly parallel to the screw axis (103) over the entire height of both sections (43, 53, 57).

2. Screw element (10) according to claim 1, **characterized in that** a guiding wall (51) is formed in the second section (57) starting from the section parting plane (54), wherein the guiding wall (51) adjoins the removal wall (50) of the first section (53) and said guiding wall (51) is increasingly spaced apart in proximal direction (101) from the insertion wall (40).

3. Screw element (10) according to claim 2, **characterized in that** the increasing spacing between the guiding wall (51) and insertion wall (40) is mainly along the circumference in removal direction (120), resulting in a wall (52) extending in the peripheral direction.

4. Screw element (10) according to any of the preceding claims, **characterized in that**, in cross-section transverse to the screw axis (103) at the level of the second section (57), the walls (51, 52, 40) have a sectional contour which corresponds approximately to a rotational slotted hole (58) and can be defined by an opening angle (55).

5. Screw element (10) according to any of the preceding claims, **characterized in that** the opening angle (55) has a maximum angle between 10° and 60°, but preferably 20° to 50°, and said angle (55) decreases towards the distal direction (102).

6. Screw element (10) according to any of the preceding claims, **characterized in that** the height of the first section (53) is greater than the height for the second section (57), that the height of the second section (57) is greater than the height of the first section (53) or that the heights of the two sections (53) and (57) are approximately equal.

7. Screw element (10) according to any of the preceding claims, **characterized in that** the tool attachment point (20) is bounded in distal direction (102) by a wall (14).

8. Screw element (10) according to claim 7, **characterized in that** the wall (14) extends as a slope in increasing distal direction (102) radially inwards.

9. Screw element (10) according to any of the preceding claims, **characterized in that** the central opening (27) has a concentric cylindrical course.

10. Screw element (10) according to any of the preceding claims, **characterized in that** the screw element (10) additionally comprises a head (11), a neck area (12) and a shaft area (13) with bone thread (18) and the tool attachment point (20) is provided in the head (11).

11. Screw element (10) according to any of the preceding claims, **characterized in that** the screw element (10) has a continuous cannula (16).

12. System of an osteosynthesis device (1), **characterized in that** the osteosynthesis device (1) consists of at least two screw elements (10) according to any of the preceding claims, wherein one screw element (10) being provided as a bone anchor with a bone thread (18) and a second screw element (2) being suitable as a locking element for fixing a connection rod (4).

13. System consisting of a screw driver (60) and at least one screw element (10), according to any of the preceding claims, **characterized in that** the screw driver (60) has a drive unit (65) with teeth (64) complementary to the first section (53), said drive unit (65) extending mainly parallel to the central axis of the screw driver (67) and the screw element (10) has guiding walls (51) in the area of the second section (57), which align the teeth (64) of the screw driver (60) rotationally round the central axis of the screw driver (67), so that a larger contact surface is provided for the teeth (64) of the screw driver (60) in the insertion direction (110) than in the removal direction (120).

## Revendications

1. Élément vis (10) pour la fixation de composants osseux et de fragments osseux composé d'une tige (13) avec un filetage extérieur (17) et un axe longitudinal (103) s'étendant le long de la tige (13) et définissant ainsi une direction distale (102) et une direction proximale (101), ainsi qu'une direction de vissage (110) et une direction de dévissage (120) opposée à celle-ci, et ayant un emplacement d'insertion d'outil (20) à partir de l'intérieur radial et l'emplacement d'insertion d'outil (20) ayant une ouverture centrale (27) et dans la paroi de cette ouverture (27) sont formés au moins cinq profils de dent orientés (21, 22, 23, 24, 25, 26) vers l'extérieur radial (104) et principalement parallèlement à l'axe de vis (103) et que, pour chaque profil de dent, on peut subdiviser une paroi de vissage (40) dans la direction de vissage (110) et une paroi de dévissage (50) dans la direction de dévissage (120), l'emplacement d'insertion d'outil (20) pouvant être divisé en au moins deux sections le long de l'axe longitudinal (103), une première section (53) étant formée en venant de la direction distale et jusqu'à la hauteur d'un plan de séparation de section (54), les surfaces des parois de vissage et de dévissage (40, 50) de chaque profil de dent (21, 22, 23, 24, 25, 26) étant à peu près de la même taille et la surface de la paroi de vissage (40) étant plus grande que la surface de la paroi de dévissage (50)
**caractérisé en ce que**
l'emplacement d'insertion d'outil (20) est ouvert dans la direction proximale (101) et débouche dans un évidement conique concentrique (15) et l'emplacement d'insertion d'outil (20) peut être divisé (53, 57) en au moins deux sections le long de la direction longitudinale (103) et la paroi de vissage (40) s'étend principalement parallèlement à l'axe de vis (103) sur toute la hauteur des deux sections (43, 53, 57).

2. Élément vis (10) selon la revendication 1, **caractérisé en ce qu'**une paroi de guidage (51) est formée dans la deuxième section (57) à partir du plan de séparation de section (54), la paroi de guidage (51) se raccordant contre la paroi de dévissage (50) de la première section (53) et cette paroi de guidage (51) étant de plus en plus éloignée de la paroi de vissage (40) dans la direction proximale (101).

3. Élément vis (10) selon la revendication 2, **caractérisé en ce que** l'espacement croissant entre la paroi de guidage (51) et la paroi de vissage (40) s'étend principalement le long de la circonférence dans la direction de dévissage (120) et qu'il en résulte une paroi (52) s'étendant dans la direction circonférentielle.

4. Élément vis (10) selon l'une quelconque des revendications précédentes, **caractérisé en ce que**, en coupe transversale à l'axe de vis (103) au niveau de la deuxième section (57), les parois (51, 52, 40) possèdent un contour de coupe qui correspond approximativement à un trou oblong de rotation (58) et qui peut être défini par un angle d'ouverture (55).

5. Élément vis (10) selon l'une quelconque des revendications précédentes, **caractérisé en ce que** l'angle d'ouverture (55) possède un angle maximal compris entre 10 ° et 60 °, mais de préférence entre 20 ° et 50 °, et cet angle (55) diminue dans la direction distale (102).

6. Élément vis (10) selon l'une quelconque des revendications précédentes, **caractérisé en ce que** la hauteur de la première section (53) est supérieure à la hauteur pour la deuxième section (57), **en ce que** la hauteur de la deuxième section (57) est supérieure à la hauteur pour la première section (53) ou **en ce que** les hauteurs des deux sections (53) et (57) sont approximativement égales.

7. Élément vis (10) selon l'une quelconque des revendications précédentes, **caractérisé en ce que** l'emplacement d'insertion d'outil (20) est délimité en direction distale (102) par une paroi (14).

8. Élément vis (10) selon la revendication 7, **caractérisé en ce que** la paroi (14) s'étend radialement vers l'intérieur sous forme de biseau dans la direction distale croissante (102).

9. Élément vis (10) selon l'une quelconque des revendications précédentes, **caractérisé en ce que** l'ouverture centrale (27) présente un développement cylindrique concentrique.

10. Élément vis (10) selon l'une quelconque des revendications précédentes, **caractérisé en ce que** l'élément vis (10) présente en outre une tête (11), une zone de col (12) et une zone de tige (13) avec un filetage osseux (18) et l'emplacement d'insertion d'outil (20) est prévu dans la tête (11).

11. Élément vis (10) selon l'une quelconque des revendications précédentes, **caractérisé en ce que** l'élément vis (10) possède une canulation continue (16).

12. Système d'un dispositif d'ostéosynthèse (1), **caractérisé en ce que** le dispositif d'ostéosynthèse (1) est constitué d'au moins deux éléments vis (10) selon l'une quelconque des revendications précédentes, un élément vis (10) étant prévu comme ancrage osseux avec un filetage osseux (18) et un deuxième élément vis (2) étant adapté en tant qu'élément de fermeture pour la fixation d'une tige de liaison (4).

13. Système composé d'un instrument de tournevis (60) et d'au moins un élément vis (10), selon l'une quelconque des revendications précédentes, **caractérisé en ce que** l'instrument de tournevis (60) possède une unité d'entraînement (65) avec des dents (64), formée de manière complémentaire à la première section (53), qui s'étend principalement parallèlement à l'axe central (67) du tournevis et l'élément vis (10) possède, dans la zone de la deuxième section (57), des parois de guidage (51) qui alignent les dents (64) de l'instrument de tournevis (60) en rotation autour de l'axe central (67) du tournevis, de sorte qu'une plus grande surface de contact est prévue pour les dents (64) de l'instrument de tournevis (60) dans la direction de vissage (110) que dans la direction de dévissage (120).
